# EUROPÄISCHE PATENTANMELDUNG

(11) **EP 1 058 085 A2**
(43) Veröffentlichungstag der Anmeldung: **06.12.2000**
(21) Anmeldenummer: 00110796.0
(22) Anmeldetag: 20.05.2000
(51) Int. Cl.: G01B 5/04

(54) **Vorrichtung und Verfahren zum Messen des Dehn- oder Kontraktionsverhaltens von fadenförmigem Prüfgut**

(30) Priorität: 29.05.1999 DE 19924637
(71) Anmelder: TEXTECHNO HERBERT STEIN GMBH & CO: KG, 41066 Mönchengladbach (DE)
(72) Erfinder: Stein, Wolfgang, Dr., 41239 Mönchengladbach (DE); Mörschel, Ulrich, Dr., 52441 Linnich (DE)
(74) Vertreter: Dallmeyer, Georg, Dipl.-Ing.

(57) **Zusammenfassung**

Bei einer Vorrichtung zum Messen des Dehn- oder Kontraktionsverhaltens von fadenförmigem Prüfgut (10), mit einem Gehäuse (2), mit mindestens zwei Fadenlieferwerken (4,8), zwischen denen eine Prüfstrecke (12) für das fadenförmige Prüfgut (10) verläuft, mit einer Kraftmeßeinrichtung (14), die die auf das fadenförmige Prüfgut (10) in der Prüfstrecke (12) ausgeübte Kraft mißt, und mit einer Heizeinrichtung (18), die das fadenförmige Prüfgut (10) in der Prüfstrecke (12) umgibt und auf eine vorgegebene Temperatur aufheizt, ist vorgesehen, daß eine Positioniereinrichtung (24) das fadenförmige Prüfgut (10) relativ zu der Heizeinrichtung (18) oder die Heizeinrichtung (18) relativ zu dem fadenförmigen Prüfgut (10) derart verschiebt, daß das Prüfgut (10) in einer Endposition außerhalb und in der anderen Endposition innerhalb der Heizeinrichtung (18) angeordnet ist.

## Beschreibung

Die Erfindung betrifft eine Vorrichtung zum Messen des Dehn- oder Kontraktionsverhaltens von fadenförmigem Prüfgut nach dem Oberbegriff des Anspruchs 1, sowie ein Meßverfahren nach dem Oberbegriff des Anspruchs 16.

Derartige Prüfgeräte sind z.B. unter der Bezeichnung "Dynafil" der Firma Textechno bekannt. Bei diesen Prüfgeräten wird das fadenförmige Prüfgut mit einstellbarer, während der Prüfung konstanter Dehnung oder Überlieferung (Verkürzung) oder ohne Längenänderung durch ein geschlossenes Heizrohr gefördert, wobei die sich einstellende Fadenzugkraft kontinuierlich gemessen wird. Je nach den eingestellten Prüfbedingungen und der Art des Prüfgutes lassen sich verschiedene Prüfverfahren realisieren:
- Dehnkraftprüfung vororientierter oder vollverstreckter Garne,
- Kräuselkraftprüfung texturierter Garne, oder
- Schrumpfkraftprüfung glatter wie texturierter Garne.

Ein anderes bekanntes Prüfgerät - TYT der Firma Lawson Hemphill - mißt kontinuierlich die Längenänderung eines unter konstanter Fadenzugkraft stehenden Fadens beim Durchlauf durch ein geschlossenes Heizrohr. Diese Prüfung wird vorzugsweise zur Bestimmung der Kräuselkontraktion texturierter Garne eingesetzt.

Die bekannten Geräte besitzen eine Prüfstrecke, die von einem Eingangs- und einem Ausgangs-Fadenlieferwerk sowie dem dazwischen angeordneten Heizrohr und der Kraftmeßeinrichtung (DYNAFIL) bzw. der Vorrichtung zur Konstanthaltung der Fadenzugkraft (TYT) gebildet wird. Die Liefergeschwindigkeiten der beiden Fadenlieferwerke können gemeinsam sowie relativ zueinander variiert werden.

Die Schmelztemperatur der üblicherweise derartigen Prüfungen unterzogenen Faserstoffe liegt bei maximal 260°C (Polyester, Nylon 6.6.). Daher muß die Heizrohrtemperatur der bekannten Prüfgeräte immer unterhalb dieses Grenzwertes eingestellt werden. Andernfalls würde der Faden beim Stillstand des Gerätes oder auch bei langsamem Lauf sowie beim Einführen eines neuen Fadens in die Prüfstrecke im Heizrohr schmelzen und zerreißen.

Wegen dieser Begrenzung der Heizrohrtemperatur sind auch die maximal möglichen Fadengeschwindigkeiten, bei denen der Faden noch die für die Prüfung erforderliche Durchwärmung, erfährt, begrenzt. Je nach Fadenfeinheit können 100 bis 200 m/min erreicht werden.

Ausgehend von einem solchen Stand der Technik liegt der Erfindung die Aufgabe zugrunde, Prüfvorrichtungen der eingangs genannten Arten derart zu verbessern, daß einerseits wesentlich höhere Prüfgeschwindigkeiten möglich sind und andererseits auch hochtemperaturbeständiges Prüfgut untersucht werden kann.

Zur Lösung dieser Aufgabe dienen die Merkmale des Anspruchs 1 bzw. 16.

Die Erfindung sieht in vorteilhafter Weise vor, daß eine Positioniereinrichtung das fadenförmige Prüfgut relativ zu der Heizeinrichtung oder die Heizeinrichtung relativ zu dem fadenförmigen Prüfgut derart verschiebt, daß das Prüfgut in einer Endposition in der Heizeinrichtung verläuft und in der anderen Endposition außerhalb der Heizeinrichtung verläuft.

Die Heizeinrichtung ist dazu nicht wie bei bisher bekannten Prüfgeräten als geschlossenes Rohr ausgebildet. Vielmehr verfügt sie über einen Heizkanal mit einem seitlichen auch automatisch zu öffnenden und zu schließenden Schlitz zum Ein- oder Ausfahren des Prüfgutes.

Eine derartige Positioniereinrichtung in Verbindung mit der Heizeinrichtung ermöglicht Heiztemperaturen weit oberhalb der Schmelztemperaturen der Faserstoffe, z.B. bis 800°C, und damit weitaus höhere Fadengeschwindigkeiten, z.B. bis 1000 m/min, bei denen der Faden die optimale Durchwärmung erreicht. Sofern der Fadenlauf gestoppt werden oder ein neuer Faden in die Prüfstrecke eingelegt werden soll, wird zunächst mit Hilfe der Positioniereinrichtung der - noch mit hoher Geschwindigkeit laufende - Faden aus der Heizeinrichtung ausgeführt. Beim erneuten Start wird umgekehrt der Faden zunächst außerhalb der Heizeinrichtung auf Prüfgeschwindigkeit gebracht und erst dann in den Heizkanal eingeführt. Auf diese Weise ist ausgeschlossen, daß das Fadenmaterial in der Heizeinrichtung schmilzt und zerreißt.

Ein weiterer Vorteil eines solchen Hochtemperaturheizeinrichtung besteht darin, daß damit auch die Prüfung hochtemperaturbeständiger Faserstoffe ermöglicht wird. Die Prüfung derartiger Materialien in den bisher bekannten Geräten mit relativ niedrigen Heiztemperaturen liefert dagegen keine verwertbaren Prüfergebnisse.

Die Fadenlieferwerke der erfindungsgemäßen Prüfvorrichtung können aus motorisch angetriebenen Galetten oder Riemchen- oder Klemmwalzenlieferwerken bestehen.

Bei einer Weiterbildung der Erfindung ist vorgesehen, daß zusätzliche Meßeinrichtungen in der Prüfstrecke zwischen dem eingangsseitigen und dem ausgangsseitigen Fadenlieferwerk, in Fadenlaufrichtung vor dem eingangsseitigen Fadenlieferwerk oder hinter dem ausgangsseitigen Fadenlieferwerk angeordnet sind. Diese zusätzlichen Meßeinrichtungen dienen dazu, Reibungsmessungen, Messungen von Filamentbrüchen, Messungen von Verwirbelungspunkten, Messungen der Fadenungleichmäßigkeit oder der Garnfeinheit durchzuführen.

Bei einem bevorzugten Ausführungsbeispiel ist vorgesehen, daß in Fadenlaufrichtung vor dem ersten Fadenlieferwerk eine Einrichtung zum Einstellen und Konstanthalten einer vorbestimmten Vorspannkraft im laufenden Prüfgut angeordnet ist, die von dem in der Prüfstrecke aufgenommenen Meßsignal der Kraftmeßeinrichtung steuerbar ist. Diese Einrichtung gewährleistet eine exakt einstellbare und während der Prüfung konstante Vorspannkraft.

In Fadenlaufrichtung hinter der Heizeinrichtung kann ein Temperatursensor zur Bestimmung der tatsächlich erreichten Fadentemperatur angeordnet sein. Dieser Temperatursensor liefert ein Meßsignal, das zur Temperatursteuerung der Heizeinrichtung verwendet werden kann.

In Fadenlaufrichtung hinter der Heizeinrichtung kann desweiteren eine Drallvorrichtung zur Erzeugung eines Falschdrahts im Prüfgut angeordnet sein.

Mit der erfindungsgemäßen Vorrichtung werden die verschiedenen Prüfverfahren der bisher bekannten Prüfgeräte in einem Gerät vereinigt. Bei der Messung am laufenden Faden ist eine Prüfung mir konstanter Dehnung oder Verkürzung des Prüfgutes und gleichzeitiger Messung der sich im Prüfgut einstellenden Kraft möglich. Alternativ kann die Prüfung mit einer auf das Prüfgut einwirkenden konstanten Zugkraft durchgeführt werden, wobei die Geschwindigkeit zumindest eines der beiden Fadenlieferwerke kontinuierlich nachgeregelt wird. Die Längenänderung des Prüfgutes, die zu jedem Zeitpunkt der Geschwindigkeitsdifferenz zwischen den beiden Fadenlieferwerken entspricht, wird kontinuierlich gemessen.

Bei Heiztemperaturen unterhalb der Schmelztemperatur des Prüfgutes ist auch eine Prüfung des stehenden Fadens möglich. Dabei wird - nach Einführen des Fadens in die Prüfeinrichtung - entweder ohne Längenänderung oder mit während der Prüfung konstanter Längenänderung des Fadens die Kraft in Abhängigkeit von der Zeit oder bei konstant gehaltener Kraft die Längenanderung in Abhängigkeit von der Zeit gemessen.

Ein wesentlicher Vorteil der Erfindung bei den Prüfungen am ruhenden Faden besteht darin, daß eine nachfolgende Messung sehr schnell durch Nachführen eines neuen Fadenabschnittes mittels der Fadenlieferwerke erfolgen kann.

Im folgenden werden unter Bezugnahme auf die Zeichnungen Ausführungsbeispiel der Erfindung näher erläutert:

Es zeigen:
- Fig. 1: eine Teilansicht des erfindungsgemäßen Prüfgerätes, und
- Fig. 2: eine Einrichtung zum Einstellen einer Vorspannung, und
- Fig. 3: ein zweites Ausführungsbeispiel.

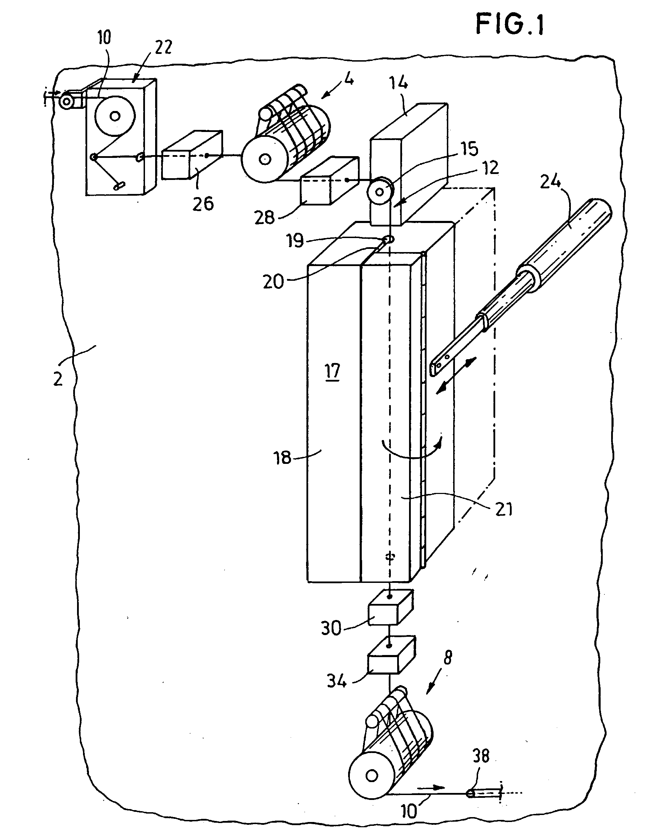

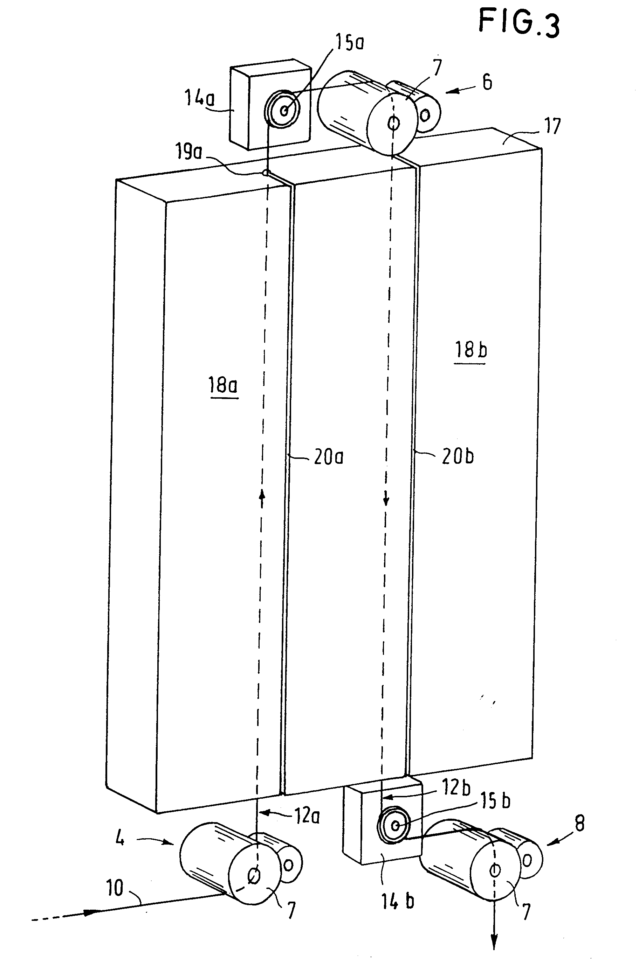

Fig. 1 zeigt ein Prüfgerät für Zugkraft- oder Längenänderungsprüfungen an fadenförmigem Prüfgut 10, das in einem Gehäuse 2 angeordnet ist. Das Prüfgut 10 kann bei stehendem oder laufendem Faden untersucht werden. Das Prüfgerät weist mindestens zwei Fadenlieferwerke 4,8 auf, die, wie aus Fig. 1 hervorgeht, aus Galetten bestehen können oder aus Riemchen- oder Klemmwalzenlieferwerken 7. Klemmwalzenlieferwerke 7 sind in Fig. 3 dargestellt. Jedes Fadenlieferwerk 4,8 wird von einem in der Drehzahl variabel ansteuerbaren Präzisionsmotor mit Drehzahlmessung (z.B. Inkrementalgeber, Resolver oder Tachogenerator) angetrieben.

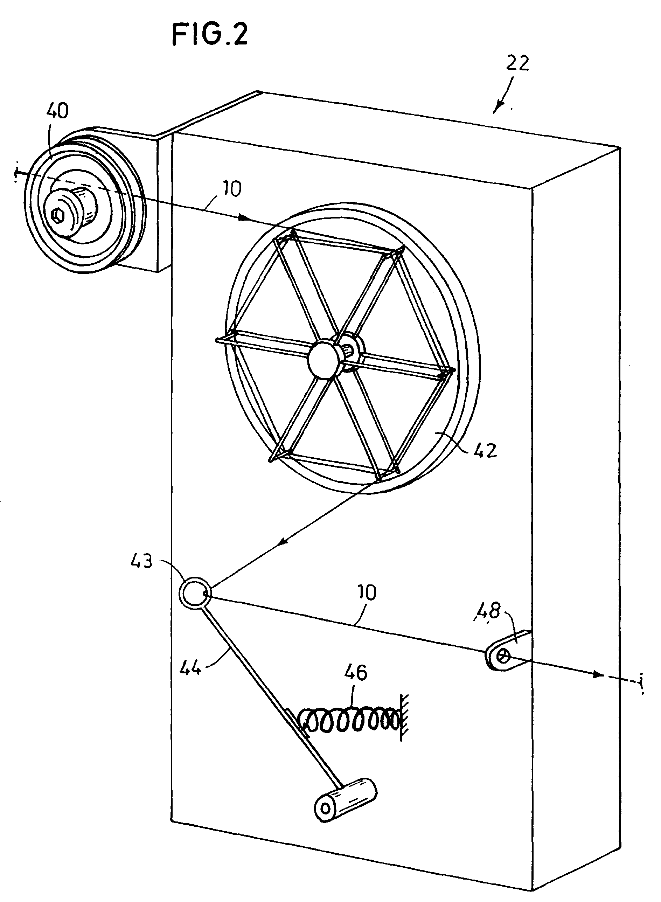

Vor dem eingangsseitigen Fadenlieferwerk 4 ist eine in Fig. 2 dargestellte Einrichtung 22 zum Einstellen und Konstanthalten einer vorbestimmten Vorspannkraft im laufenden Prüfgut 10 angeordnet. Diese Einrichtung 22 ist pneumatisch oder elektronisch verstellbar, wobei die Einrichtung von dem am Prüfgut 10 aufgenommenen Meßsignal der Kraftmeßeinrichtung 14 steuerbar ist. Die Einstellung der Einrichtung 22 erfolgt bei gleicher Geschwindigkeit der beiden Fadenlieferwerke 4,8, d.h. ohne Verzug, bis die von der Kraftmeßeinrichtung 14 gemessene Kraft der einzustellenden Vorspannkraft entspricht.

Die Einrichtung 22 besteht aus einer eingangsseitigen Tellerbremse 40, über die das Prüfgut 10 z.B. von einem Spulenträger zugeführt wird. Das Prüfgut 10 wird auf einer Haspel 42 aufgewickelt, wobei sich mindestens zwei komplette Windungen auf der Haspel 42 befinden. Von der motorisch angetriebenen Haspel 42 wird das Prüfgut durch eine Öse 43 eines Tasthebels 44 hindurchgeführt und über eine ausgangsseitige Führungsöse 48 einer Meßeinrichtung 26 bzw. dem eingangsseitigen Fadenlieferwerk 4 zugeführt. Der Tasthebel 44 ist mit einer Druckfeder 46 vorgespannt. Die Federkraft der Druckfeder 46 ist manuell bzw. elektronisch in Abhängigkeit eines Steuersignals einstellbar, so daß eine gewünschte Vorspannkraft in dem Prüfgut 10 einstellbar ist. Die Stellung des Tasthebels 44 steuert den Motorantrieb der Haspel 42, so daß eine exakt konstante Vorspannkraft auf das Prüfgut 10 einwirkt.

Alternativ zu der Einrichtung 22 kann vor Beginn einer Prüfung zunächst bei laufendem Faden das Verhältnis der Geschwindigkeiten des eingangsseitigen und des ausgangsseitigen Fadenlieferwerks 4,8 so verändert werden, daß die Zugkraft in der Prüfstrecke 12 den gewünschten Wert für die Vorspannkraft erreicht. Von dem so gefundenen Geschwindigkeitsverhältnis ist auszugehen, wenn die Zugkraft oder die Längenänderung des Prüfgutes 10 für die eigentliche Messung eingestellt wird.

Zwischen den Fadenlieferwerken 4,8 verläuft eine Prüfstrecke 12 für das fadenförmige Prüfgut 10. In der Prüfstrecke 12 ist eine Kraftmeßeinrichtung 14 angeordnet, mit der die im Prüfgut 10 herrschende Zugkraft gemessen werden kann. Die Längenänderungen des Prüfgutes 10 in der Prüfstrecke 12 werden aus den Geschwindigkeitsunterschieden der Fadenlieferwerke 4,8 ermittelt. Das Prüfgut wird in der Prüfstrecke über eine an der Kraftmeßeinrichtung 14 befestigte Fadenleitrolle 15 umgelenkt und tritt darauffolgend in einen Heizkanal 19 einer Heizeinrichtung 18 ein. Auf der dem Gehäuse 2 abgewandten Seite des Heizkanals 19 ist ein Schlitz 20 in dem Gehäuse 17 der Heizeinrichtung 18 vorgesehen, durch den das Prüfgut 10 aus dem Heizkanal 19 herausgeführt werden kann. So ist bei dem Ausführungsbeispiel der Fig. 1 vorgesehen, daß die Heizeinrichtung 18 in das Gehäuse 2 mit Hilfe einer Positioniereinrichtung 24 zumindest teilweise versenkbar ist, wodurch das Prüfgut 10 durch Zurückziehen der Heizeinrichtung vollständig aus dem Heizkanal 19 herausgeführt werden kann.

Das Gehäuse 17 der Heizeinrichtung 18 weist eine mechanisch betätigte schwenkbare Klappe 21 auf, die in den vollständig eingefahrenen oder vollständig ausgefahrenen Zustand der Heizeinrichtung 18 geschlossen ist, und die automatisch geöffnet wird, sobald die Heizeinrichtung 18 verfahren werden soll. In der Schließstellung vermeidet die schwenkbare Klappe 21 zu hohe Wärmeverluste.

Alternativ ist es auch möglich, bei am Gehäuse 2 ortsfest angeordneter Heizeinrichtung 18 die Fadenlieferwerke 4,8 einschließlich der Meßeinrichtung 14 parallel zu verschieben bis die Prüfstrecke 12 außerhalb des Heizkanals 19 verläuft.

Nach einer weiteren Alternative können die Heizeinrichtung 18 und die Fadenlieferwerke 4,8 einschließlich der Meßeinrichtung 14 ortsfest an dem Gehäuse 2 befestigt sein, wobei nur das Prüfgut 10 in der Prüfstrecke 12 mit Hilfe von Fadenführungseinrichtungen aus dem Heizkanal 19 herausgeleitet oder in diesen eingeführt wird.

Dadurch, daß das Prüfgut 10 mit Hilfe der Positioniereinrichtung 24 schnell in den Heizkanal 19 eingebracht oder aus diesem herausgenommen werden kann, ist es möglich, die Heizeinrichtung 18 mit Temperaturen bis zu einer Obergrenze von 800°C und mehr zu betreiben. Dies ermöglicht Prüfgeschwindigkeiten von über 1000 m/min. Dabei ist die Heiztemperatur stufenlos ab Raumtemperatur auf eine gewünschte Temperatur im Heizkanal 19 einstellbar. Weiterhin können Prüfungen an hochtemperaturbeständigen Fadenmaterialien durchgeführt werden.

In Fig. 1 sind zusätzliche Meßeinrichtungen 26,28 dargestellt, die vor, in oder hinter der Prüfstrecke 12 angeordnet sein können. Diese zusätzlichen Meßeinrichtungen 26,28 dienen dazu, Reibungsmessungen, Messungen von Filamentbrüchen, Messungen von Verwirbelungspunkten, Messungen des Bauschvolumens texturierter Garne, Messungen der Fadenungleichmäßigkeit oder der Garnfeinheit durchzuführen.

In Fadenlaufrichtung hinter der Heizeinrichtung 18 ist ein Temperatursensor 30 am unteren Ende des Heizkanals 19 vorgesehen, mit dem die erreichte Fadentemperatur gemessen werden kann. Das Temperatursignal kann zur Heizungssteuerung und/oder zur Steuerung der Transportgeschwindigkeit der Fadenlieferwerke 4,8 verwendet werden.

In Fadenlaufrichtung hinter der Heizeinrichtung 18 kann auch eine Dralleinrichtung 34 angeordnet sein, die einen Falschdraht im Prüfgut 10 erzeugt.

Hinter dem ausgangsseitigen Fadenlieferwerk 8 kann das Prüfgut 10 mit Hilfe einer Absaugeinrichtung 38 abgeführt und gesammelt werden.

Mit dem beschriebenen Prüfgerät können beispielsweise folgende Messungen durchgeführt werden:

### Messung unter konstanter Krafteinwirkung auf das Prüfgut

In diesem Fall wird eine vorbestimmte konstante Zugkraft mit Hilfe von variierenden Geschwindigkeitsverhältnissen der Fadenlieferwerke 4,8 eingestellt und die dabei entstehende Dehnung oder Kontraktion des Prüfgutes unter Einwirkung einer vorbestimmten Temperatur gemessen. Die Dehnungsmessungen erfolgen durch eine Drehzahldifferenzmessung zwischen den Fadenlieferwerken 4,8.

### Messung der Zugkraft bei konstanter Längenänderung des Prüfgutes

In diesem Fall wird eine vorbestimmte Dehnung oder Verkürzung über eine unterschiedliche Transportgeschwindigkeit der Fadenlieferwerke 4,8 fest eingestellt. Die sich jeweils ergebende Zugkraft wird gemessen und aufgezeichnet, wobei die Messungen mit unterschiedlichen Temperaturen im Heizkanal und mit unterschiedlichen Tränsportgeschwindigkeiten durchgeführt werden können.

### Dynamische Kraft-Dehnungs-Prüfungen

Die Geschwindigkeitsverhältnisse der beiden Fadenlieferwerke können während der Prüfung auch programmgesteuert verändert werden. Auf diese Weise sind dynamische Kraft-Dehnungs-Kurven meßbar, indem beispielsweise die Dehnung stufenweise oder kontinuierlich gesteigert und die sich dabei ergebende (Dehn-) Kraft gemessen und in Abhängigkeit von der Dehnung aufgezeichnet wird.

Dabei wird die Dehnung des Prüfgutes 10, wie bereits erwähnt, über die unterschiedliche Transportgeschwindigkeit der Fadenlieferwerke 4,8 eingestellt.

### Kraft- oder Längenänderungsmessung mit steigender Fadengeschwindigkeit des Prüfgutes

Zur Ermittlung von geeigneten Einstellparametern hinsichtlich der Temperatur im Heizkanal 19 und der Fadengeschwindigkeit für die vorgenannten Messungen können für Voruntersuchungen des Dehnungs- und Kontraktionsverhaltens des Prüfgutes 10 Kraft- oder Längenänderungsmessungen mit steigender Fadengeschwindigkeit und konstanter Temperatur im Heizkanal 19 durchgeführt werden.

Fig. 3 zeigt ein weiteres Ausführungsbeispiel, bei dem das Prüfgut 10 über ein erstes Lieferwerk 4 einer ersten Prüfstrecke 12a zuführbar ist, die in einer ersten Heizeinrichtung 18a in einem Heizkanal 19a verläuft. An Ende der ersten Prüfstrecke 12a wird das Prüfgut 10 über eine Fadenleitrolle 15a einer ersten Kraftmeßeinrichtung 14a, die der ersten Prüfstrecke zugeordnet ist, einem zweiten Lieferwerk 6 zugeführt, über das das Prüfgut 10 in eine zweite Prüfstrecke 12b hineingeführt wird. Die zweite Prüfstrecke 12b verläuft in einem Heizkanal 19b einer zweiten Heizeinrichtung 18b. Die zweite Prüfstrecke 12b enthält eine zweite Kraftmeßeinrichtung 14b, an der das Prüfgut 10 über eine Fadenleitrolle 15b, einen dritten Lieferwerk 8 zugeführt wird.

Die Lieferwerke 4,6,8 dieses Ausführungsbeispiels sind entweder Galetten, Riemchenlieferwerke oder Klemmwalzenlieferwerke 7. Beide Heizeinrichtungen 18a,18b weisen jeweils einen Schlitz 20a,20b auf, über den das Prüfgut 10 in den Heizkanal 19a,19b einführbar ist. Es versteht sich, daß die Schlitze 20a,20b wie bei dem Ausführungsbeispiel der Fig. 1 verschließbar gestaltet sein können. So können die Schlitze 20a,20b beispielsweise durch eine parallel verschiebbare oder schwenkbare Klappe entweder separat oder gemeinsam verschließbar sein.

Die Vorrichtung gemäß der Fig. 3 ist insbesondere für das Verstrecken von Filamentkabel oder grober Fadenstränge von 10.000 bis 20.000 dtex geeignet.

Dabei kann ein Vorverstrecken des Prüfgutes 10 in der ersten Prüfstrecke 12a und ein Endverstrecken in der zweiten Prüfstrecke 12b erfolgen.

Auch bei diesem Ausführungsbeispiel kann alternativ das Gehäuse 17 mit den Heizeinrichtungen 18a,18b in das Gehäuse 2 versenkt werden oder alternativ das fadenförmige Prüfgut 10 aus der Heizeinrichtung 18a,18b parallelverschoben herausgeführt werden.

## Patentansprüche

1. Vorrichtung zum Messen des Dehn- oder Kontraktionsverhaltens von fadenförmigem Prüfgut (10), mit einem Gehäuse (2), mit mindestens zwei Fadenlieferwerken (4,6,8), zwischen denen eine Prüfstrecke (12;12a,12b) für das fadenförmige Prüfgut (10) verläuft, mit einer Kraftmeßeinrichtung (14;14a,14b), die die auf das fadenförmige Prüfgut (10) in der Prüfstrecke (12) ausgeübte Kraft mißt, und mit einer Heizeinrichtung (18;18a,18b), die das fadenförmige Prüfgut (10) in der Prüfstrecke (12;12a,12b) umgibt und auf eine vorgegebene Temperatur aufheizt,
**dadurch gekennzeichnet,**
daß eine Positioniereinrichtung (24) das fadenförmige Prüfgut (10) relativ zu der Heizeinrichtung (18;18a,18b) oder die Heizeinrichtung (18;18a,18b) relativ zu dem fadenförmigen Prüfgut (10) derart verschiebt, daß das Prüfgut (10) in einer Endposition außerhalb und in der anderen Endposition innerhalb der Heizeinrichtung (18;18a,18b) angeordnet ist.

2. Vorrichtung nach Anspruch 1, dadurch gekennzeichnet, daß die Obergrenze der Heiztemperatur der Heizeinrichtung (18) in einem Bereich von über 260°C bis 800°C einstellbar ist.

3. Vorrichtung nach Anspruch 1 oder 2, dadurch gekennzeichnet, daß die Heizeinrichtung (18;18a,18b) einen sich längs erstreckenden Heizkanal (19;19a,19b) zur Aufnahme des Prüfgutes (10) aufweist und daß das Gehäuse (17) der Heizeinrichtung (18;18a,18b) einen Schlitz (20;20a,20b) zum Einführen des Prüfgutes (10) in den Heizkanal (19;19a,19b) aufweist.

4. Vorrichtung nach Anspruch 3, dadurch gekennzeichnet, daß der Schlitz (20;20a,20b) verschließbar ist.

5. Vorrichtung nach einem der Ansprüche 1 bis 4, dadurch gekennzeichnet, daß zwischen dem eingangsseitigen Fadenlieferwerk (4) und dem ausgangsseitigen Fadenlieferwerk (8) oder in Fadenlaufrichtung vor dem eingangsseitigen Fadenlieferwerk (4) oder in Fadenlaufrichtung hinter dem ausgangsseitigen Fadenlieferwerk (8) zusätzliche Meßeinrichtungen (26,28) angeordnet sind.

6. Vorrichtung nach Anspruch 5, dadurch gekennzeichnet, daß die zusätzlichen Meßeinrichtungen aus Meßeinrichtungen zur Reibungsmessung oder zur Messung von Filamentbrüchen, von Verwirbelungspunkten, des Bauschvolumens texturierter Garne, der Fadenungleichmäßigkeit oder der Garnfeinheit bestehen.

7. Vorrichtung nach einem der Ansprüche 1 bis 6, dadurch gekennzeichnet, daß in Fadenlaufrichtung vor den eingangsseitigen Fadenlieferwerk (4) eine Einrichtung (22) zum Einstellen und Konstanthalten einer vorbestimmten Vorspannkraft im laufenden Prüfgut (10) angeordnet ist, die von dem Meßsignal der Kraftmeßeinrichtung (14) steuerbar ist.

8. Vorrichtung nach einem der Ansprüche 1 bis 7, dadurch gekennzeichnet, daß in Fadenlaufrichtung hinter der Heizeinrichtung (18;18a,18b) ein Temperatursensor (30) zur Bestimmung der erreichten Fadentemperatur angeordnet ist.

9. Vorrichtung nach einem der Ansprüche 1 bis 8, dadurch gekennzeichnet, daß in Fadenlaufrichtung hinter der Heizeinrichtung (18;18a,18b) eine Drallvorrichtung (34) angeordnet ist, die einen Falschdraht im Prüfgut (10) erzeugt.

10. Vorrichtung nach einem der Ansprüche 1 bis 9, dadurch gekennzeichnet, daß die Transportgeschwindigkeit des eingangsseitigen Fadenlieferwerks (4;6) und des ausgangsseitigen Fadenlieferwerks (6;8) programmgesteuert unterschiedlich einstellbar sind.

11. Vorrichtung nach Anspruch 10, dadurch gekennzeichnet, daß durch kontinuierliches Verändern des Transportgeschwindigkeitsverhältnisses der Fadenlieferwerke (4,6,8) eine konstante Fadenzugkraft im Prüfgut einstellbar ist, wobei das Transportgeschwindigkeitsverhältnis von dem Meßsignal der Kraftmeßeinrichtung (14) bei gleichzeitiger Messung des Transportgeschwindigkeitsverhältnisses steuerbar ist.

12. Vorrichtung nach Anspruch 10, dadurch gekennzeichnet, daß durch feste Vorgabe des Transportgeschwindkeitsverhältnisses der Fadenlieferwerke (4,6,8) eine konstante Längenänderung des Prüfgutes (10) bei gleichzeitiger Messung der Zugkraft einstellbar ist.

13. Vorrichtung nach Anspruch 12, dadurch gekennzeichnet, daß zur Ermittlung einer dynamischen Kraft-Dehnungs-Kurve die auf das Prüfgut (10) einwirkende Dehnung programmgesteuert bei gleichzeitiger Messung der Zugkraft veränderbar ist.

14. Vorrichtung nach einem der Ansprüche 1 bis 13, dadurch gekennzeichnet, daß die Temperatur der Heizeinrichtung (18) während der Zugkraft- oder Längenänderungsprüfungen programmgesteuert veränderbar ist.

15. Vorrichtung nach einem der Ansprüche 1 bis 14, dadurch gekennzeichnet, daß drei Fadenlieferwerke (4,6,8) vorgesehen sind, zwischen denen zwei Prüfstrecken (12a,12b) für das fadenförmige Prüfgut (10) angeordnet sind, wobei jede Prüfstrecke (12a,12b) durch eine separate Heizeinrichtung (18a,18b) verläuft.

16. Vorrichtung nach Anspruch 15, dadurch gekennzeichnet, daß jeder Prüfstrecke (12a,12b) eine separate Kraftmeßeinrichtung (14a,14b) zugeordnet ist.

17. Verfahren zum Messen des Dehn- oder Kontraktionsverhaltens von fadenförmigem Prüfgut (10), indem das fadenförmige Prüfgut (10) in einer Prüfstrecke (12;12a,12b) in einer Heizeinrichtung (18;18a,18b) auf eine vorbestimmte Temperatur aufgeheizt wird und einer vorbestimmten Zugkraft oder einer vorbestimmten Dehnung oder Verkürzung oder keiner Längenänderung ausgesetzt wird, wobei die entstehenden Dehnungen und Kräfte in dem Prüfgut (10) gemessen werden, gekennzeichnet durch
- Einstellen einer Vorspannkraft in dem Prüfgut (10),
- Verschieben des laufenden oder stehenden Prüfgutes (10) relativ zu der Heizeinrichtung (18,18a,18b) oder Verschieben der Heizeinrichtung relativ zu dem Prüfgut (10), so daß das Prüfgut (10) in die bereits vorgeheizte oder noch auf Raumtemperatur befindliche und erst anschließend aufzuheizende Heizeinrichtung (18;18a,18b) gelangt,
- Durchführen einer Zugkraft- oder Längenänderungsprüfung,
- durch Messen der Längenänderung des Prüfgutes (10) bei konstanter auf das Prüfgut (10) einwirkender Zugkraft, oder
- durch Messen der Zugkraft bei konstant eingestellter Dehnung des Prüfgutes (10).

18. Verfahren nach Anspruch 17, dadurch gekennzeichnet, daß eine konstante Dehnung des Prüfgutes (10) über ein festes Transportgeschwindigkeitsverhältnis der Fadenlieferwerke (4,6,8) eingestellt wird und die Zugkraft gemessen wird.

19. Verfahren nach Anspruch 18, dadurch gekennzeichnet, daß zur Messung dynamischer Kraft-Dehnungs-Kurven die Dehnungswerte schrittweise oder kontinuierlich verändert werden.

20. Verfahren nach einem der Ansprüche 17 bis 19, dadurch gekennzeichnet, daß eine konstante Zugkraft auf das Prüfgut (10) über ein variables Transportgeschwindigkeitsverhältnis der Fadenlieferwerke (4,6,8) eingestellt wird und die Dehnung gemessen wird, wobei das Transportgeschwindigkeitsverhältnis durch das Kraftmeßsignal gesteuert wird.

21. Verfahren nach Anspruch 17 bis 19, gekennzeichnet durch das Aufteilen der Prüfstrecke (12) in zwei separate nacheinander von dem Prüfgut (10) durchlaufene Prüfstrecken (12a,12b), die jeweils unterschiedlich beheizbar sind, wobei in der ersten Prüfstrecke (12a) ein Vorverstrecken des Prüfgutes und in der zweiten Prüfstrecke (12b) ein Endverstrecken des Prüfgutes (10) erfolgt.
